(19) Europäisches Patentamt
European Patent Office
Office européen des brevets

(11) Veröffentlichungsnummer: **0 301 411 B1**

(12) **EUROPÄISCHE PATENTSCHRIFT**

(45) Veröffentlichungstag der Patentschrift: **15.04.92**

(51) Int. Cl.^5: **G02F 1/35**

(21) Anmeldenummer: **88111764.2**

(22) Anmeldetag: **21.07.88**

# (54) Film aus mindestens einer monomolekularen Schicht.

(30) Priorität: **25.07.87 DE 3724688**
**04.02.88 DE 3803224**

(43) Veröffentlichungstag der Anmeldung:
**01.02.89 Patentblatt 89/05**

(45) Bekanntmachung des Hinweises auf die Patenterteilung:
**15.04.92 Patentblatt 92/16**

(84) Benannte Vertragsstaaten:
**BE CH DE FR GB LI NL SE**

(56) Entgegenhaltungen:
**EP-A- 0 149 754**
**EP-A- 0 250 099**

(73) Patentinhaber: **HOECHST AKTIENGESELLSCHAFT**
**Postfach 80 03 20**
**W-6230 Frankfurt am Main 80(DE)**

(72) Erfinder: **Laschewsky, André, Dr.**
**Pfuhlgasse 13**
**W-5400 Koblenz(DE)**
Erfinder: **Ringsdorf, Helmut, Prof. Dr.**
**Kehlweg 41**
**W-6500 Mainz(DE)**
Erfinder: **Interthal, Werner, Dr.**
**Dr. Ludwig-Opel-Strasse 62**
**W-6090 Rüsselsheim(DE)**
Erfinder: **Lupo, Donald, Dr.**
**Am Holderbusch 28**
**W-6239 Eppstein/Taunus(DE)**
Erfinder: **Prass, Werner, Dr.**
**Am Mühlbach 16**
**W-6500 Mainz(DE)**
Erfinder: **Scheunemann, Ude, Dr.**
**Loreleistrasse 1**
**W-6230 Frankfurt am Main 80(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung bezieht sich auf einen Film aus mindestens einer monomolekularen Schicht, welcher für Zwecke der nichtlinearen Optik brauchbar ist.

Die nichtlineare Optik (NLO) hat eine große Bedeutung für die zukünftige Entwicklung der Informationstechnik, wegen ihres Potentials für schnelle Signalverarbeitung und -Übertragung und für neue Methoden in der Datenverarbeitung Spezielle organische Verbindungen haben höhere Wirkungsgrade und schnellere Schaltzeiten als die herkömmlichen anorganischen Substanzen für nichtlineare Optik.

Eine Substanz hat nichtlinear-optische Eigenschaften, wenn die Polarisation $\vec{P}$ die durch die Wechselwirkung zwischen der Substanz und einem starken elektrischen Feld (einem Laserstrahl oder einem starken Gleichspannungsfeld) erzeugt wird, von höheren Potenzen der Feldstärke abhängt, nach Gleichung (1):

$$\vec{P} = \chi^{(1)} \cdot \vec{E}_1 + \chi^{(2)} : \vec{E}_1\vec{E}_2 + \chi^{(3)} \vdots \vec{E}_1\vec{E}_2\vec{E}_3 + \ldots \qquad (1)$$

$\chi^{(1)}$, $\chi^{(2)}$, $\chi^{(3)}$ sind die sogenannten Suszeptibilitäten 1., 2., und 3. Ordnung. E ist das elektrische Feld, das Komponenten mehrerer Frequenzen enthalten kann. Durch NLO-Wechselwirkungen können Felder mit neuen Frequenzen erzeugt sowie die Brechungsindizes des Materials geändert werden. Die Suszeptibilitäten $\chi^{(2)}$ und $\chi^{(3)}$ hängen von den sogenannten molekularen Hyperpolarisierbarkeiten $\beta$ und $\gamma$ ab.

Wichtige nichtlinear-optische Effekte, die von $\chi^{(2)}$ abhängen, sind die Frequenzverdopplung eines Lichtstrahls, insbesondere eines Laserstrahls, die parametrische Verstärkung eines schwachen Lichtsignals und die elektrooptische Umwandlung von elektrischen Signalen. Um Effekte 2. Ordnung zu erzielen, müssen die aktiven Moleküle nichtzentrosymmetrisch ausgerichtet werden, da für zentrosymmetrische Substanzen $\chi^{(2)} = 0$.

Ein Verfahren, das eine für die NLO besonders günstige geordnete Ausrichtung herstellen kann, ist das Langmuir-Blodgett (LB) Verfahren. Bei diesem Verfahren werden Moleküle auf einer Wasseroberfläche gespreitet, durch Verkleinerung der Fläche pro Molekül parallel angeordnet, und bei konstantem Schub durch Ein- und Austauchen eines Trägers auf ein Substrat aufgezogen. Pro Tauchgang wird eine monomolekulare Schicht unter Erhaltung ihrer Ordnung übertragen. Für den Aufbau von LB-Schichten verwendet man amphiphile Moleküle, d.h. Moleküle, die ein hydrophiles Ende (einen "Kopf") und ein hydrophobes Ende (einen "Schwanz") haben.

Um LB-schichten mit hohen Suszeptibilitäten 2. Ordnung zu erzielen, stellt man organische Verbindungen her, die sowohl hohe molekulare Hyperpolarisierbarkeiten 2. Ordnung $\beta$ als auch amphiphile Eigenschaften haben. Eine Verbindung hat einen großen Wert für $\beta$, wenn sie ein konjugiertes Elektronensystem (z.B. einen Benzolring) enthält, in das eine oder mehrere Elektronendonorgruppen und eine oder mehrere Elektronenakzeptorgruppen eingebaut werden. Am Donor- oder Akzeptorende wird eine hydrophobe Gruppe eingebaut. Die Hyperpolarisierbarkeit wird vergrößert, wenn das Molekül Licht im Wellenlängenbereich des einstrahlenden elektrischen Feldes oder des durch die NLO erzeugten Feldes absorbiert (sogenannte Resonanzverstärkung). Allerdings sind Absorptionen für viele Anwendungen unerwünscht, da sie Verluste verursachen und die optische Stabilität (die Lichtintensität, die ohne permanente Materialstörungen ertragen werden kann) negativ beeinflussen. Eine ideale Verbindung hat eine große Hyperpolarisierbarkeit, ohne im gewünschten Wellenlängenbereich stark zu absorbieren.

Es wurde gefunden, daß bestimmte amphiphile Farbstoffe diese Eigenschaften besitzen.

Somit betrifft die Erfindung den in den Ansprüchen beschriebenen Film.

Der erfindungsgemäße Film besteht aus mindestens einer monomolekularen Schicht, welche eine amphiphile Verbindung der Formel (I) enthält oder aus dieser besteht.

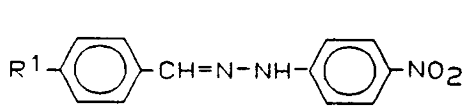

(I),

worin $R^1$ den Rest

$$CH_3-(CH_2)_m-O- \quad ,$$
$$CH_3-(CH_2)_n-\underset{\overset{\|}{O}}{C}-O- \quad ,$$
$$CH_3 \ (CH_2)_n\underset{|}{N}(CH_2)_lH \text{ oder}$$
$$CH_3 \ (CH_2)_n(CO)\underset{|}{N}(CH_2)_lH$$

bedeutet und m eine Zahl von 10 bis 25, vorzugsweise 15 bis 19, n eine Zahl von 8 bis 22, vorzugsweise 10-14 ist und 1 eine Zahl von 0-25, vorzugsweise 0-4 oder 14-22 ist.

Die Moleküle der Verbindung der Formel (I) sind in der Schicht gleichsinnig, parallel nebeneinander, nichtzentrosymmetrisch orientiert. In vielen Fällen stehen sie in wesentlichen senkrecht zur Schichtebene, was von Vorteil ist.

Zum Aufbau dickerer Filme unter Erhalt der Nichtzentrosymmetrie besteht der erfindungsgemäße Film aus mindestens zwei monomolekularen Schichten unterschiedlicher Zusammensetzung. Benachbarte Schichten weisen in den meisten Fällen jeweils unterschiedliche Zusammensetzung auf. Die unterschiedliche Zusammensetzung ist jedoch keine Grundvoraussetzung. Beispielsweise wechselt jeweils eine Schicht, welche eine Verbindung der Formel (I) enthält oder aus dieser besteht, ab mit einer Schicht, welche keinen Farbstoff der Formel I aber eine andere amphiphile Verbindung insbesondere ohne Chrompophor enthält, oder aus dieser besteht. Schichten, die aus einer Verbindung des Formel (I) bestehen, können noch geringe Mengen Salze oder der beim Spreiten verwendete Subphase enthalten.

Falls $A^1$, $A^2$, $A^3$ verschiedene Amphiphile ohne Chromophor und $F^1$, $F^2$, $F^3$ verschiedene Farbstoffe der Formel I bedeuten, so lassen sich verschiedene Möglichkeiten des Schichtaufbaues schematisch wie folgt darstellen:

$F^1/F^2/F^1/F^3$
$A^1/F^1/A^1/F^1$
$A^1/F^1/A^2/A^2$
$F^1/A^1/A^2/A^3$
$F^1/F^1/F^1/F^1$
$F^1/F^2/F^1/F^2$
$F^1/F^2/A^1/A^2$
$F^1 + A^1/F^2/F^1/F^3$
$A^1/F^1 + A^1/A^1/F^2 + A^2$
$A^1 + F^1/A^1/A^2/A^3$
$A^1 + F^1/A^2 + F^2/A^1 + F^1/A^2 + F^2$

Amphiphile Verbindungen enthalten gleichzeitig mindestens eine hydrophile und mindestens eine hydrophobe Gruppe im Molekül.

Der hydrophobe Teil der zweiten amphiphilen Verbindung soll eine gewisse Mindestlänge aufweisen. Es ist bevorzugt, wenn die zweite amphiphile Verbindung mindestens einen hydrophoben Teil, in dem mindestens 8 Kohlenstoffatome enthalten sind, und mindestens eine der folgenden polaren Gruppen: Äther-, Hydroxy-, Carbonsäure-, Carbonsäureester-, Amin-, Carbonsäureamid-, Ammoniumsalz-, Sulfat-, Sulfonsäure-, Phosphorsäure-, Phosphonsäure-, Phosphonsäureester-, Phosphonsäureamid-, Phosphorsäureester- oder Phosphorsäureamid-Gruppe enthält.

Besonders bevorzugt ist es, wenn die amphiphile Verbindung aus mindestens einem hydrophoben Teil mit mindestens 8 Kohlenstoff-Atomen und mindestens einem polaren Teil besteht, der ausgewählt ist aus den folgenden Gruppen

$-OR^5$

$-COOR^3$

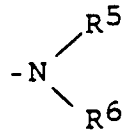

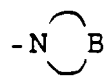

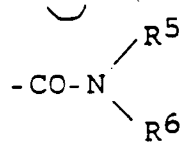

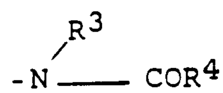

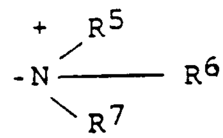

$-SO_3H$

$-OSO_3R^3$

$-OPO(OR^3)(OR^4)$

-E

-O-E

$-NR^3$-E,

wobei $R^3$ bis $R^7$, B und E folgendes bedeuten:

| | |
|---|---|
| $R^3$ und $R^4$ | unabhängig voneinander H oder Cl-$C_3$-Alkyl, |
| $R^5$, $R^6$ und $R^7$ | unabhängig voneinander H, $C_1$-$C_4$-Alkyl, $-C_2H_4OH$ oder $-CH_2-CHOH-CH_3$, insbesondere H oder $CH_3$ |
| B | einen zweiwertigen organischen Rest, so daß 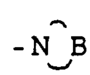 einen stickstoffhaltigen Heterocyclus, insbesondere einen 5- oder 6-gliedrigen, gesättigten oder ungesättigten Heterocyclus mit 1 bis 3 C-Atomen oder N- und O-Atomen oder N- und S-Atomen bildet, und |
| E | |

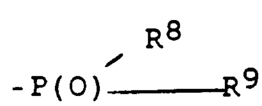 oder

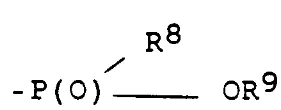,

wobei $R^8$ und $R^9$ unabhängig voneinander für

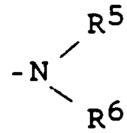

stehen.

Beispielsweise kann die amphiphile Verbindung eine Fettsäure der allgemeinen Formel II

$CH_3(CH_2)_mCO_2H$ (II)

sein,
wobei m eine Zahl von 8 bis 25, vorzugsweise 12 bis 22, bedeutet.

Vorteilhafterweise wird als amphiphile Verbindung ein ungesättigtes Säureamid der allgemeinen Formel III

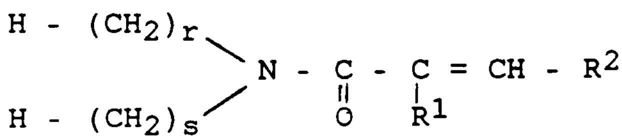
(III)

eingesetzt, in der
$R^1$ H, Cl, F, CN oder $(CH_2)_tH$,
$R^2$ H, $(CH_2)_uH$ oder $-CH=CH-(CH_2)_uH$,
r und s unabhängig voneinander eine Zahl von 0 - 22 und
t und u unabhängig voneinander eine Zahl von 0 - 24 insbesondere 0-18, bedeuten.
r ist vorzugsweise eine Zahl von null bis 18 und s ist vorzugsweise null.

Üblicherweise erfolgt die Herstellung der Filme und der monomolekularen Schichten mittels LB-Technik auf Wasseroberflächen. Daher ist es bevorzugt, wenn die amphiphile Verbindung nur eine geringe Wasserlöslichkeit, insbesondere eine Wasserlöslichkeit von weniger als 5 g/l bei 20 °C aufweist.

Um eine ausreichend geringe Wasserlöslichkeit dieser amphiphilen Verbindung zu erzielen, ist es bevorzugt, daß mindestens einer der Werte für r, s, t und u mindestens 10 beträgt. Es ist jedoch nicht nötig, daß sämtliche Werte mindestens 10 betragen. Eine besonders bevorzugte Möglichkeit besteht darin, daß einer der Werte für t und u mindestens 10 und der andere Wert maximal 1 beträgt. Eine andere besonders bevorzugte Möglichkeit besteht darin, daß der Wert für r mindestens 10 beträgt und die Werte für t und u maximal 1 betragen.

Es ist auch möglich als amphiphile Verbindung gesättigte Säureamide der allgemeinen Formel IV

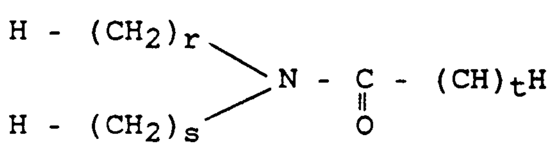
(IV)

einzusetzen, wobei r und s unabhängig voneinander eine Zahl von 0 - 22 und t eine Zahl von 0 - 24 bedeutet, und mindestens einer der Werte für r, s und t mindestens 8 beträgt.

Auch hier ist es zur Erzielung einer ausreichenden Hydrophobie sinnvoll, daß mindestens einer der Werte für r, s und t mindestens 10 beträgt. Eine besonders bevorzugte Möglichkeit besteht darin, daß die Werte für r und s maximal 1 und der Wert für t mindestens 10 betragen. Eine andere besonders bevorzugte Möglichkeit besteht darin, daß der Wert für r mindestens 10 und der Wert für t maximal 1 beträgt.

Es ist günstig, wenn die Kettenlänge des eingesetzten amphiphilen Farbstoffs und der zweiten amphiphilen Verbindung aufeinander abgestimmt werden. Es ist daher bevorzugt, daß die Länge der Alkykette des eingesetzten amphiphilen Phenylhydrazons (Farbstoff) und des hydrophoben Teils der zweiten amphiphilen Verbindung sich um maximal 1 nm unterscheiden. Falls die zweite amphiphile

Verbindung zwei oder mehr hydrophobe Teile aufweisen sollte, so ist der längste hydrophobe Teil maßgebend.

Zur Herstellung der erfindungsgemäßen Filme werden die Verbindungen der Formel I und/oder die chromophor-freien amphiphilen Verbindungen in einer Langmuir-Blodgett-Filmwaage in einem hochflüchtigen Lösungsmittel auf die Oberfläche der Subphase gebracht (gespreitet). Das Lösungsmittel verdampft und die Verbindungen bleiben auf der Oberfläche zurück. Aus der Abmessung der Oberfläche, dem Spreitvolumen und der Konzentration der Lösung wird die mittlere Flache pro Molekül berechnet.

Die gespreiteten Moleküle werden mit einer Barriere zusammengeschoben, wobei die Ketten bei wachsender Dichte im wesentlichen senkrecht zur Grenzschicht orientiert werden. Phasenübergänge bei der Kompression der Moleküle lassen sich in der Schub-Flächen-Isotherme erkennen. Phasenzustände lassen sich in der Schub-Flächen-Isotherme erkennen. Während der Kompression entsteht durch Selbstorganisation der Moleküle in der Grenzschicht ein hochgeordneter, monomolekularer Film, dessen konstante Schichtdicke durch die Kettenlänge der Moleküle bestimmt wird. Die typische Dicke eines solchen Films liegt zwischen 2-3 nm, was etwa der Länge der längsten Moleküle in der Schicht entspricht.

Je nach der Hydrophilie des Substrats (= Träger) werden die hydrophilen oder die hydrophoben Enden der aufgetragenen amphiphilen Moleküle (einschließlich des Farbstoffs) dem Substrat zugewandt.

Die zunächst auf der Wasseroberfläche gebildete monomolekulare Schicht wird durch Ein- oder Austauchen eines sauberen geeigneten Trägers unter Erhalt der molekularen Ordnung von der Wasseroberfläche abgenommen. Durch Wiederholung dieses Verfahrens lassen sich mehrere monomolekulare Schichten nacheinander auftragen und so die Dicke des erhaltenen Films variieren.

Als Träger sind geeignet Festkörper mit sauberen Oberflächen, wie beispielsweise Glas, Keramik- oder Metallplatten, Kunststoffschichten aus beispielsweise PMMA, Polystyrol, Polycarbonat, Polyethylen, Polypropylen oder Polytetrafluorethylen, oder Metallschichten auf den genannten Substraten. Als Träger lassen sich ferner Metallfolien verwenden. In diesem Fall lassen sich die nichtlinear-optischen Eigenschaften allerdings nur in Reflexion beobachten.

Um den Wert der Suszeptibilität 2. Ordnung ($\chi^{(2)}$) experimentell zu bestimmen, verwendet man meist das Phänomen der Frequenzverdopplung, bei der in einer aktiven Substanz eingestrahltes Licht mit Frequenz $\omega$ in Licht mit Frequenz $2\omega$ umgewandelt wird:

Der erfindungsgemäße Film ergibt, insbesondere wenn er alternierend Schichten aus Verbindungen der Formel (I) und aus anderen amphiphilen Verbindungen enthält, eine stabile Multischicht mit guten nichtlinear-optischen Eigenschaften. Er ist dadurch geeignet beispielsweise für elektrooptische Schalter, Diodenlaser-frequenzverdoppler oder optische parametrische Verstärker, z. B. als sogenannte Auffrischer schwacher Lichtsignale in optischen Signalübertragungsnetzen.

Für spezielle Anwendungen, z.B. den Aufbau planarer nichtlinearer Lichtwellenleiter unter Anwendung von Licht mit bestimmter Polarisation, ist es von Vorteil, wenn die Chromophore des Farbstoffs (sogenannte aktive Gruppen) im wesentlichen senkrecht zur Oberfläche des Substrats (des Trägers der Schicht) orientiert sind. Bei einigen Farbstoffen ergibt sich aber, daß die Chromophore im wesentlichen parallel zur Oberfläche orientiert sind, obwohl sich die hydrophoben langen Alkylketten im wesentlichen senkrecht ausrichten.

Dies gilt auch für die oben beschriebenen Farbstoffe der Formel (I), mit einer Acyloxy-phenylgruppe.

Es wurde gefunden, daß sich eine im wesentlichen senkrechte Orientierung der Chromophore in diesen Farbstoffen dadurch erreichen läßt, daß die Farbstoffe in Mischungen mit anderen amphiphilen Substanzen in monomolekularen orientierten Schichten aufgezogen werden.

Dabei ist es nötig, daß hinsichtlich der Konzentration der anderen amphiphilen Verbindungen bestimmte Konzentrationen beachtet werden. Falls ihre Menge unter 10 Mol-% liegt, wurde kein oder nur ein schwacher Aufrichtungseffekt beobachtet.

Eine bevorzugte Ausgestaltung der Erfindung betrifft daher einen Film, bestehend aus mindestens einer monomolekularen Schicht, die neben dem amphiphilen Phenylhydrazon der allgemeinen Formel Ia

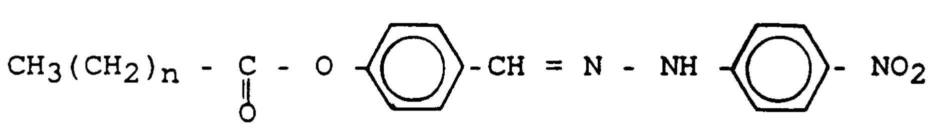

(Ia)

noch mindestens eine zweite amphiphile Verbindung in einem Molanteil von 10 bis 90 % enthält. Dabei betragt die Summe aller amphiphilen Verbindungen 100 %. Vorzugsweise werden dabei Phenylhydrazone verwendet, in deren allgemeiner Formel n die Werte von 10 - 14 annimmt.

Besonders deutlich tritt der beabsichtigte Effekt auf, wenn der Molanteil der zweiten amphiphilen

Verbindung 40 - 75 % beträgt.

Auch der auf den Träger aufgetragene erfindungsgemäße Film, dessen Schichten Farbstoffe der Formel Ia mit Acyloxy-Gruppen und amphiphile Verbindungen enthalten, ist stabil und weist gute nichtlinearoptische Eigenschaften auf. Die Moleküle sind parallel und gleichsinnig, d.h. nicht zentrosymmetrisch angeordnet. Durch den Zusatz dieser amphiphilen Verbindung lassen sich Filme gewinnen, in denen nicht nur der hydrophile Teil, sondern auch das Chromophor der Farbstoffmoleküle senkrecht zur Schichtebene ausgerichtet ist.

Die Farbstoffe der allgemeinen Formel I lassen sich wie folgt erhalten:

1) Ether
Alkylbromid + p-Hydroxybenzaldehyd→ p-Alkoxybenzaldehyd
p-Alkoxybenzaldehyd + p-Nitro-phenylhydrazin→ entsprechendes Nitrophenylhydrazon
2) Ester
Carbonsäurechlorid + p-Hydroxybenzaldehyd→ p-Acyloxy-benzaldehyd
p-Acyloxy-benzaldehyd + p-Nitro-phenylhydrazin→ entsprechendes Nitrophenylhydrazon.
3) Mono alkylamine
Carbonsäure + Anilin→Acylanilin.
Acylanilin + Lithiumalanat→Monoalkylanilin.
Monoalkylanilin + Dimethylformamid/Phosphoroxychlorid→ p-Monoalkylamino-benzaldehyd.
p-Monoalkylamino-benzaldehyd + p-Nitrophenylhydrazin→ entsprechendes Nitrophenylhydrazon.
4) Dialkylamine
Überschuß an Alkylbromid + Anilin→N,N-Dialkylanilin
Einführung einer Aldehydgruppe mit Dimethylformamid(=DMF)/Phosphoroxychlorid→p-Dialkylamino-benzaldehyd.
p-Dialkylamino-benzaldehyd +p-Nitrophenylhydrazin→ entsprechendes Nitrophenylhydrazon.
5) Amid
p-Monoalkylamino-benzaldehyd (vgl. 3.) + Carbonsäurechlorid→p-(N-Acyl-N-alkylamino)-benzaldehyd.
Hieraus mittels p-Nitrophenylhydrazin das entsprechende Nitrophenylhydrazon.
6) Dialkylamin mit verschiedenen Alkylgruppen
Alkylanilin + Carbonsäurechlorid→ N-Alkyl-N-acyl-anilin. Reduktion mit Lithiumalanat→ p-(N-Alkyl$^1$-N-Alky$^2$ amino)-benzaldehyd.
Daraus mittels p-Nitrophenylhydrazin das entsprechende Nitrophenylhydrazon.

Die folgenden Beispiele erläutern die Erfindung.

**Beispiel 1**

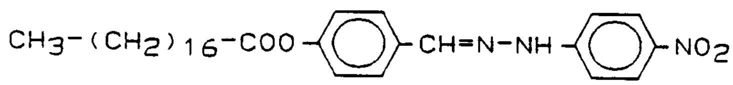

6,11 g 4-Hydroxybenzaldehyd wurden in 200 $cm^3$ trocknem Methylenchlorid gelöst, 8 $cm^3$ Triethylamin zugesetzt und die Lösung im Eisbad auf 5 °C abgekühlt. Bei dieser Temperatur wurden 15,1 g Stearinsäurechlorid, gelöst in 100 $cm^3$ trockenem Methylenchlorid, innerhalb von 20 Minuten zugetropft. Anschließend wurde die Reaktionslösung mit 1 m HCl, Wasser, 5 %iger $Na_2CO_3$-Lösung und wieder mit Wasser gewaschen, die organische Phase mit Natriumsulfat getrocknet und das Lösemittel im Vakuum abgezogen. Zur Reinigung wurden das Produkt noch zweimal aus Methanol umkristallisiert.
Schmelzbereich: 59,5 - 60,5 °C Ausbeute: 12,74 g
3,89 g des Stearinsäureesters wurden zu einer Lösung von 1,53 g 4-Nitrophenylhydrazin in einer Mischung von 10 $cm^3$ Eisessig, 10 $cm^3$ Wasser und 100 $cm^3$ Ethanol gegeben. Die Mischung wurde zwei Stunden bei Raumtemperatur gerührt, der organgefarbene Niederschlag abgesdaugt und mehrmals aus Methanol, Ethylacetat und Aceton umkristallisiert.
Schmelzbereich: 131 - 134 °C Ausbeute: 1,28 g

**Beispiel 2**

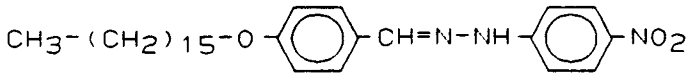

11,6 g Kaliumcarbonat wurden 2 Stunden bei 800 °C geglüht und nach dem Abkühlen in eine Lösung von 9,77 g 4-Hydroxybenzaldehyd, 24,43 g 1-Bromhexadecan und 100 mg Kaliumiodid in 600 $cm^3$ trocknem Aceton suspendiert. Die Suspension wurde unter Feuchtigkeitsausschluß 60 Stunden unter Rückfluß gekocht und anschließend heiß filtriert. Zum Filtrat wurden 600 $cm^3$ Hexan gegeben und die Lösung mit 10 %iger $Na_2CO_3$-Lösung und Wasser gewaschen. Die organische Phase wurde mit Natriumsulfat getrocknet und das Lösemittel im Vakuum abgezogen. Das Produkt wurde anschließend noch einmal aus Methanol umkristallisiert.

Schmelzbereich: 51 - 59 °C Ausbeute: 14,7 g

3,47 g des Hexadecylethers wurden zu einer Lösung von 1,53 g 4-Nitrophenylhydrazin in einer Mischung von 10 $cm^3$ Eisessig, 10 $cm^3$ Wasser und 100 $cm^3$ Ethanol gegeben und die Mischung 2 Stunden lang bei Raumtemperatur gerührt. Der orangefarbene Niederschlag wurde abgesaugt und mehrmals aus Ethylacetat umkristallisiert.

Schmelzbereich: 115 - 117 °C Ausbeute 3,16 g

**Beispiel 3**

Ein Siliziumplättchen (4 cm x 1 cm) wurde aus einem Siliziumwafer herausgeschnitten und folgendermaßen gereinigt:

1) 1 Stunde Behandlung in einem Ultraschallbad in einer Mischung aus einem Teil 30 %igen $H_2O_2$ und vier Teilen konzentrierter Schwefelsäure. Anschließend mit sauberem Wasser abspülen.

2) 20 Sekunden in Ammoniumfluorid-gepufferte HF-Lösung tauchen und anschließend mit sauberem Wasser abspülen.

Nach dieser Behandlung waren die Siliziumplättchen hydrophob (Kontaktwinkel zu Wasser: 75 °C).

Schichten von Acrylsäureoctadecylamid wurden auf das Siliziumplättchen nach dem Verfahren von Langmuir und Blodgett übertragen, indem auf dem Trog einer Langmuir-Filmwaage 0,1 $cm^3$ einer $10^{-4}$-molaren Lösung von Acrylsäureoctadecylamid in n-Hexan auf einer wäßrigen Subphase gespreitet wurden. Durch Verkleinerung der monofilmbedeckten Wasseroberfläche wurde der Schub auf 25 mN/m eingeregelt und bei desem Wert konstant gehalten (Flächenbedarf bei diesem Schub: 0,21 $nm^2$/Molekül). Das Siliziumplättchen wurde nun senkrecht von oben durch die Wasseroberfläche in den Trog der Filmwaage eingetaucht (Eintauchgeschwindigkeit: 20 mm/min) und nach einer kurzen Pause (10 Sekunden) am unteren Umkehrpunkt wieder herausgenommen (Austauchgeschwindigkeit: 10 mm/min). Sowohl bei Ein- als auch beim Austauchvorgang wurde eine Monolage auf das Siliziumplättchen übertragen. Durch Wiederholung des Tauchvorgangs und durch Variation der Eintauchtiefen wurden auf einen Träger so 10, 20, 30, 40 und 50 Schichten übertragen. Mittels Ellipsometermessungen wurden anschließend die Schichtdicken und der Brechungsindex der LB-Filme gemessen. (Ergebnis: Schichtdicke: 2 41 nm pro Monolage; Brechungsindex bei 633 nm: 1,51)

**Beispiel 4**

23,28 g (0,25 mol) frisch destilliertes Anilin und 167,94 g (0,55 mol) 1-Bromhexadecan wurden zusammengegeben und bei 90 °C unter Stickstoffatmosphäre 3 Tage lang gerührt. Dann wurden 700 ml Toluol zugegeben und mit 10 % $Na_2CO_3$-Lösung ausgeschüttelt. Anschließend wurde mit 5 % HCl angesäuert und das ausgefallene Hydrochlorid abfiltriert. Danach wurde wieder mit 700 ml Toluol versetzt, mit 10 % $Na_2CO_3$ ausgeschüttelt, die organische Phase mit $Na_2SO_4$ getrocknet und das Lösungsmittel unter reduziertem Druck entfernt. Nach zweimaligem Umkristallisieren aus Ethanol wurden 56,5 g (42 % d.Th.) farblose Kristalle erhalten, die bei 48-49 °C schmelzen.

Eine Aufschlämmung von 10 g (18,5 mol) N,N-Dihexadecylanilin in 20 g frisch destilliertem DMF wurde auf 5 °C abgekühlt und innerhalb von 5 Minuten mit 2,8 g (18,5 mmol) $POCl_3$ versetzt. Nach beendeter Zugabe wurde eine Stunde bei 20 °C und 3 Stunden bei 80 °C gerührt. Nach dem Abkühlen wurde die Reaktionsmischung mit 40 g Eiswasser zersetzt und mit 10 ml 5 M NaOH-Lösung neutralisiert. Der entstandene Niederschlag wurde abgesaugt und aus Methanol und Hexan umkristallisiert. Man erhielt 5,1 g (48 % d.Th.) farblose Kristalle vom Schmelzpunkt 56-57 °C.

0,1 g (0,18 mmol) N,N-Dihexadecylaminobenzaldehyd und 0,4 g (2,6 mmol) 4-Nitrophenylhydrazin wurden in einer Mischung aus 5 ml Eisessig, 3 ml Wasser und 30 ml Ethanol durch kurzes Erhitzen gelöst. Nach Stehen über Nacht wurde die entstandenen dunkelroten Kristalle abfiltriert und aus Hexan und Methanol umkristallisiert. Es wurden 50 mg (39 % d.Th.) roter Kristalle von Schmelzpunkt 47 ° C erhalten.

| Elementaranalyse: | C | H | N |
|---|---|---|---|
| berechnet: | 76,65 % | 10,86 % | 7,95 % |
| gefunden: | 76,14 % | 10,78 % | 7,80 % |

**Beispiel 5**

Ein Siliziumplättchen wurde wie in Beispiel 3 gereinigt und nach dem Langmuir-Blodgett Verfahren analog zu Beispiel 3 mit 10, 20, 40 und 60 Monolagen des Farbstoffs aus Beispiel 2 beschichtet (Subphase: Wasser; Temperatur: 20 ° C; Schub: 35 mN/m; Flächenbedarf 0,26 $nm^2$/Molekül; Eintauchgeschwindigkeit: 20 mm/min; Austauchgeschwindigkeit: 10 mm/min). Die Schichten wurden wie in Beispiel 3 ellipsometrisch untersucht. Die daraus bestimmte Schichtdicke betrug 2,75 nm pro Monolage und der Brechungsindex bei 633 nm war 1,58.

**Beispiel 6**

Auf einem Siliziumplättchen wurden, wie in Beispiel 5, 10, 20, 40 und 60 Monolagen des nach Beispiel 1 synthetisierten Farbstoffs übertragen und ellipsometrisch Schichtdicke und Brechungsindex bei 633 nm bestimmt. Die Schichtdicke pro Monolage betrug 2,75 nm und der Brechungsindex war 1,58.

**Beispiel 7**

Ein Glas-Objektträger (76 mm x 26 mm) wurde nach folgendem Verfahren gereinigte:
Das Glas wurde eine Stunde lang in einer Mischung aus einem Teil 30 %igem Wasserstoffperoxid und vier Teilen konzentrierter Schwefelsäure ultrabeschallt. Danach wurde der Objektträger mit Wasser abgespült und 15 Minuten bei 50 ° C in einer Reinigerlösung ( 2-4 g/l) ultrabeschallt. Abschließend wurde wieder gründlich mit sauberem Wasser abgespült.
Der so behandelte Objektträger wurde in einer Langmuir-Filmwaage in die wäßrige Subphase eingetaucht, der in Beispiel 1 synthetisierte Farbstoff an der Wasseroberfläche gespreitet, komprimiert und der Glasträger aus der Subphase herausgezogen (Subphase: Wasser; Temperatur: 20 ° C; Schub: 35 mN/m; Flächenbedarf: 0,24 $nm^2$/Molekül; Austauchgeschwindigkeit: 10 mm/min). Dabei wurde auf Vorder- und Rückseite der Glasplatte je eine Monoschicht übertragen.
Die beschichtete Glasplatte wurde in die Probenkammer einer Apparatur zur Bestimmung der nichtlinear optischen Suszeptibilitäten eingespannt und gemessen. Die Suszeptibilität 2. Ordnung betrug 0,56 x $10^{-6}$ esu und die nichtlineare Polarisation 108 x $10^{-30}$ esu.

Die Apparatur zu Bestimmung der nichtlinear-optischen Suszeptibilität arbeitet wie folgt:
Ein Nd:YAG-Laser erzeugt einen gepulsten Laserstrahl der Wellenlänge 1064 Nanometer ($\omega$ = 9398 $cm^{-1}$), der durch einen halbdurchlässigen Spiegel in 2 Teilstrahlen aufgespalten wird. Der erste Teilstrahl wird durch Frequenzverdopplung in einer Referenzprobe in einen Referenzstrahl der Wellenlänge 532 Nanometer (2 $\omega$ = 18796 $cm^{-1}$) umgewandelt, der von einem Fotovervielfacher gemessen wird. Der zweite Teilstrahl trifft auf einen Glasträger, auf dessen beide Seiten die zu untersuchende Langmuir-Blodgett-schicht aufgezogen ist. Dabei bildet die Richtung des Laserstrahls mit der Normalen zur Glasträger-Ebene den Winkel $\theta$. Nach Bestrahlen der Langmuir-Blodgett-schicht weist der Lichtstrahl ebenfalls Licht der Frequenz 2 $\omega$ auf. Danach wird die verbleibende Grundwelle ($\omega$) von Filtern absorbiert. Die Intensität des verbleibenden (freqenzverdoppelten) Strahls wird von einem zweiten Fotovervielfacher gemessen. Die Intensität wird durch die Intensität des Referenzsignals geteilt, um Schwankungen der Laserleistung rechnerisch ausgleichen zu können. Die Abhängigkeit des normierten Signals vom Drehwinkel $\theta$ wird beobachtet bzw. errechnet. Aus der Amplitude und der Winkelabhängigkeit des Frequenzverdoppelten Signals können die Suszeptibilität zweiter Ordnung und die Orientierung der Chromophore auf der Oberfläche bestimmt werden. Die Dicke und der Brechungsindex der LB-Schicht, die ebenfalls in die Rechnung eingehen, lassen sich durch Ellipsometrie messen.

**Beispiel 8**

Ein Glas-Objektträger wurde wie in Beispiel 7 mit einer Monolage des nach Beispiel 2 synthetisierten Farbstoffs beschichtet und die Suszeptibilität sowie die nichtlineare Polarisation gemessen. Die Suszeptibilität 2. Ordnung betrug 1,7 x $10^{-6}$ esu und die nichtlinearen Polarisation 325 x $10^{-30}$ esu.

**Beispiel 9**

Ein Glas-Objektträger wurde wie in Beispiel 7 gereinigt und mit einer Monolage Acrylsäureoctadecylamid beschichtet (Subphase: Wasser; Temperatur: 20 °C; Schub: 25 mN/m; Austauchgeschwindigkeit:10 mm/min). Danach wurde der Film von der Wasseroberfläche der Filmwaage abgesaugt, der nach Beispiel 2 synthetisierte Farbstoff gespreitet und durch Eintauchen des Trägers eine Monolage Farbstoff übertragen (Schub: 35 mN/m; Eintauchgeschwindigkeit: 20 mm/min). Im Anschluß daran wurde der Farbstoff-Film von der Wasseroberfläche abgesaugt, Acrylsäureoctadecylamid gespreitet und beim Austauchen wieder eine Monolage übertragen. In dieser Art wurde fortgefahren, bis eine alternierende Schicht aus 3 Lagen Acrylamid und 2 Lagen Farbstoff aufgebaut war. An dieser Schicht wurde wie in Beispiel 7 die Suszeptibilität 2. Ordnung gemessen. Ihr Wert betrug 0,27 x $10^{-6}$ esu.

**Beispiel 10**

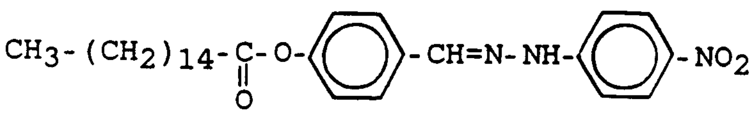

6,11 g 4-Hydroxybenzaldehyd wurden in 200 ml trockenem Methylenchlorid gelöst, 8 ml Triethylamin zugesetzt und die Lösung im Eisbad auf 5 °C abgekühlt. Bei dieser Temperatur wurden 13,7 g Palmitinsäurechlorid, gelöst in 100 ml trockenem Methylenchlorid, innerhalb von 30 Minuten zugetropft. Anschließend wurde die Reaktionslösung mit 1 m Salzsäure, Wasser, 5 % $Na_2CO_3$-Lösung und wieder mit Wasser gewaschen, die organische Phase mit Natriumsulfat getrocknet und das Lösungsmittel im Vakuum abgezogen. Zur Reinigung wurde der p-Palmitoyloxy-benzaldehyd aus Methanol umkristallisiert.
Schmelzbereich: 68 - 71 °C Ausbeute: 14,02 g
3,16 g des Palmitinsäureesters wurden zu einer Lösung von 1,53 g 4-Nitrophenylhydrazin in einer Mischung von 10 ml Eisessig, 10 ml Wasser und 100 ml Ethanol gegeben. Die Mischung wurde zwei Stunden bei Raumtemperatur gerührt, der orangefarbene Niederschlag abgesaugt und zweimal aus Methylenchlorid sowie einmal aus Methanol umkristallisiert.
Schmelzbereich: 129 - 132 °C Ausbeute: 2,51 g

**Beispiel 11**

Sechs Glas-Objektträger (76 mm x 26 mm) wurden nach folgendem Verfahren gereinigt:
Das Glas wurde eine Stunde lang in einer Mischung aus einem Teil 30 %igem Wasserstoffperoxid und vier Vol.-Teilen konzentrierter Schwefelsäure ultrabeschallt. Danach wurde der Objektträger mit Wasser abgespült und 15 Minuten bei 50 °C in einer Lösung eines alkalischen Reinigungsmittels (4 g/l Extran(R) flüssig der Merck AG) ultrabeschallt. Anschließend wurde gründlich mit sauberem Wasser gespült.

Es wurden Mischungen aus dem in Beispiel 10 synthetisierten Farbstoff und Acrylsäureoctadecylamid in den Mol-Verhältnissen 95 : 5. 90 : 10, 80 : 20, 60 : 40 und 40 : 60 hergestellt.

Für jede Mischung wurde ein gereinigter Objektträger in einer Langmuir-Filmwaage in die wäßrige Subphase eingetaucht, die Farbstoff/Acrylsäureoctadecylamid-Mischung auf der Wasseroberfläche gespreitet und komprimiert und der Träger aus der Subphase herausgezogen. Dabei wurde auf Vorder- und Rückseite der Glasplatte je eine Monoschicht übertragen. (Aufziehbedingungen: Subphase Wasser, Temperatur 20 °C, Schub 30 mN/m, Austauchgeschwindigkeit 1,5 cm/min.).

Die beschichteten Glasplatten wurden in die Probenkammer einer Apparatur zur Bestimmung der nichtlinear-optischen Suszeptibilität eingespannt und wie in Beispiel 7 beschrieben, gemessen. Aus den Meßergebnissen konnte bestimmt werden, daß die Chromophore in den Schichten der Mischungen mit den Verhältnissen 95 : 5, 90 : 10 und 80 : 20 (Farbstoff/Acrylsäureoctadecylamid) im wesentlichen parallel zur Oberfläche liegen. Jedoch ist in Schichten der Mischungen mit 40 Mol.-% oder mehr Acrylsäureoctadecylamidanteil der Chromophor im wesentlichen senkrecht zur Oberfläche ausgerichtet.

**Beispiel 12**

Fünf Glas-Objektträger wurden wie in Beispiel 11 gereinigt. Mischungen aus dem in Beispiel 10 synthetisierten Farbstoff und Palmitinsäure wurden in den Mol-Verhältnissen 95 : 5, 90 : 10, 80 : 20, 60 : 40 und 40 : 60 hergestellt.

Für jede Mischung wurde wie in Beispiel 11(gleiche Aufziehbedingungen) eine Monoschicht auf einen Träger aufgezogen. Die beschichteten Glasplatten wurden in die Probenkammer der Apparatur zur Bestimmung der nichtlinear optischen Suszeptibilität eingespannt und gemessen. Aus den Meßergebnissen konnte bestimmt werden, daß die Chromophore in den Schichten der Mischungen mit den Mol-Verhältnissen 95 : 5, 90 :10 und 80 : 20 (Farbstoff/Palmitinsäure) im wesentlichen parallel zur Oberfläche liegen, aber in den Schichten der Mischungen mit 40 % oder mehr Palmitinsäureanteil im wesentlichen senkrecht zur Oberfläche ausgerichtet sind.

**Patentansprüche**

1. Film, bestehend aus mindestens einer monomolekularen Schicht aus einer amphiphilen Verbindung, dadurch gekennzeichnet, daß die Schicht eine Verbindung der Formel (I)

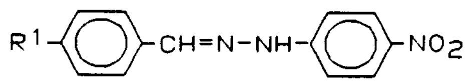

(I),

worin $R^1$ den Rest

$CH_3-(CH_2)_m-O-$ ,

$CH_3-(CH_2)_n-C(=O)-O-$ ,

$CH_3(CH_2)_nN(CH_2)_lH$ oder

$CH_3(CH_2)_n(CO)N(CH_2)_lH$ und

m eine Zahl von 10 bis 25 ,
n eine Zahl von 8 bis 22 und 1 eine Zahl von 0-25 bedeutet,
enthält oder aus dieser besteht, wobei deren Moleküle gleichsinnig, parallel nebeneinander, nichtzentrosymmetrisch orientiert sind.

2. Film nach Anspruch 1, dadurch gekennzeichnet, daß er aus mindestens zwei monomolekularen Schichten unterschiedlicher Zusammensetzung besteht, wobei jeweils eine Schicht, welche eine Verbindung der Formel (I) enthält oder aus dieser besteht, abwechselt mit einer Schicht, welche eine andere amphiphile Verbindung enthält oder aus dieser besteht.

3. Film nach Anspruch 2, dadurch gekennzeichnet, daß er aus mindestens zwei monomolekularen Schichten unterschiedlicher Zusammensetzung besteht, wobei jeweils eine Schicht, welche eine Verbindung der Formel (I) enthält oder aus dieser besteht, abwechselt mit einer Schicht, welche eine ungesättigte amphiphile Verbindung enthält oder aus dieser besteht.

4. Film nach Anspruch 3, dadurch gekennzeichnet, daß die ungesättigte amphiphile Verbindung die allgemeine Formel III

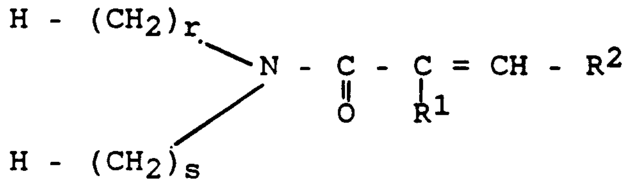

(III)

aufweist, in der
$R^1$ H, Cl, F, CN, oder $(CH_2)_tH$,
$R^2$ H, $(CH_2)_uH$ oder $-CH=CH-(CH_2)_uH$,
r und s unabhängig voneinander eine Zahl von 0-22 und
t und u unabhängig voneinander eine Zahl von 0-24 bedeuten.

**5.** Film gemäß Anspruch 1, dadurch gekennzeichnet, daß die monomolekulare Schicht noch mindestens eines zweite amphiphile Verbindung in einem Molanteil von 10 bis 90 % enthält, die Summe aller amphiphilen Verbindungen 100 % beträgt und in der Formel I $R^1$ die Bedeutung von $CH_3(CH_2)_nCO_2$- mit n = 8 - 22 hat.

**6.** Film gemäß Anspruch 5, dadurch gekennzeichnet, daß n 10 bis 14 beträgt.

**7.** Film gemäß Anspruch 5, dadurch gekennzeichnet, daß der Molanteil der zweiten amphiphilen Verbindung 40 bis 75 % beträgt.

**8.** Film nach Anspruch 5, dadurch gekennzeichnet, daß die zweite amphiphile Verbindung mindestens einen hydrophoben Teil, in dem mindestens 8 Kohlenstoffatome enthalten sind, und mindestens eine der folgenden polaren Gruppen: Äther-, Hydroxy-, Carbonsäure-, Carbonsäureester-, Amin-, Carbonsäureamid-, Ammoniumsalz-, Sulfat-, Sulfonsäure-, Phosphorsäure-, Phosphonsäure-, Phosphonsäureester-, Phosphonsäureamid-, Phosphorsäureester- oder Phosphorsäureamid-Gruppe enthält.

**9.** Film nach Anspruch 8, dadurch gekennzeichnet, daß die zweite amphiphile Verbindung aus mindestens einem hydrophoben Teil mit mindestens 8 Kohlenstoff-Atomen und mindestens einem polaren Teil besteht, der ausgewählt ist aus den folgenden Gruppen

$-OR^5$

$-COOR^3$

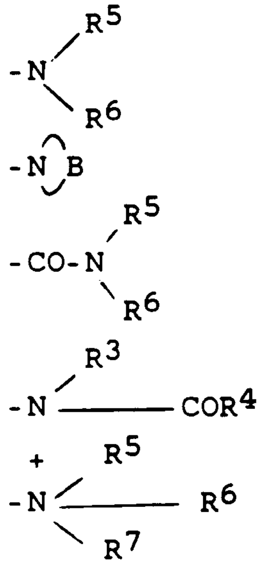

$-SO_3H$

$-OSO_3R^3$

$-OPO(OR^3)(OR^4$

$-PO(OR^3)(OR^4)$

$-E$

$-O-E$

$-NR^3-E$,

wobei $R^3$ bis $R^7$, B und E folgendes bedeuten:

| | |
|---|---|
| $R^3$ und $R^4$ | unabhängig voneinander H oder $C_1$-$C_3$-Alkyl, |
| $R^5$, $R^6$ und $R^7$ | unabhängig voneinander H, $C_1$-$C_4$-Alkyl, $-C_2H_4OH$, oder $-CH_2-CHOH-CH_3$, insbesondere H oder $CH_3$ |
| B | einen zweiwertigen organischen Rest, so daß 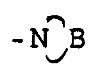 einen stickstoffhaltigen Heterocyclus, insbesondere einen 5- oder 6-gliedrigen gesättigten oder ungesättigten Heterocyclus mit 1 bis 3 C-Atomen oder N- und O-Atomen oder N- und S-Atomen bildet, und |
| E | 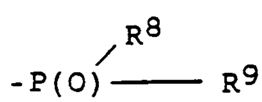 oder 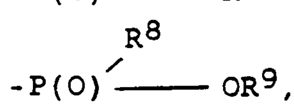, |

wobei $R^8$ und $R^9$ unabhängig voneinander für

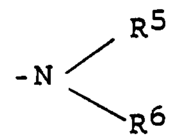

stehen.

**10.** Film nach Anspruch 9, dadurch gekennzeichnet, daß die zweite amphiphile Verbindung eine Fettsäure der allgemeinen Formel II

$CH_3(CH_2)_mCO_2H$ (II)

ist,
wobei m eine Zahl von 8 bis 25, vorzugsweise 12 bis 22 bedeutet.

**11.** Film nach Anspruch 8, dadurch gekennzeichnet, daß die zweite amphiphile Verbindung die allgemeine Formel III

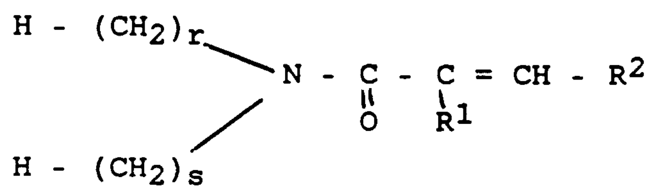

(III)

aufweist, in der
$R^1$ H, Cl, F, CN oder $(CH_2)_tH$,
$R^2$ H, $(CH_2)_uH$ oder $-CH=CH-(CH_2)_uH$,
r und s unabhängig voneinander eine Zahl von 0-22 und
t und u unabhängig voneinander eine Zahl von 0-24 bedeuten.

**12.** Film gemäß Anspruch 11, dadurch gekennzeichnet, daß mindestens einer der Werte für r, s, t und u mindestens 10 beträgt.

**13.** Film gemäß Anspruch 12, dadurch gekennzeichnet, daß einer der Werte für t und u mindestens 10 und der andere Wert maximal 1 beträgt.

**14.** Film gemäß Anspruch 12, dadurch gekennzeichnet, daß der Wert für r mindestens 10 beträgt und die Werte für t und u maximal 1 betragen.

**15.** Film gemäß Anspruch 8, dadurch gekennzeichnet, daß die zweite amphiphile Verbindung die allgemeine Formel IV

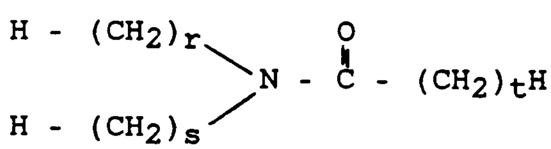

(IV)

aufweist, wobei r und s unabhängig voneinander eine Zahl von 0-22 und t eine Zahl von 0-24 bedeutet, und mindestens einer der Werte für r, s und t mindestens 8 beträgt

**16.** Film gemäß Anspruch 15, dadurch gekennzeichnet, daß mindestens einer der Werte für r, s und t mindestens 10 beträgt.

**17.** Film gemäß Anspruch 16, dadurch gekennzeichnet, daß die Werte für r und s maximal 1 und der Wert für t mindestens 10 betragen.

**18.** Film gemäß Anspruch 16, dadurch gekennzeichnet, daß der Wert für r mindestens 10 und der Wert für t maximal 1 beträgt.

**19.** Film gemäß Anspruch 8, dadurch gekennzeichnet, daß die Länge der Alkylkette des eingesetzten amphiphilen Phenylhydrazons und des hydrophoben Teils der zweiten amphiphilen Verbindung sich um maximal 1 nm unterscheiden.

**20.** Film nach Anspruch 5, dadurch gekennzeichnet, daß die zweite amphiphile Verbindung eine Wasserlöslichkeit bei 20 °C von weniger als 5 g/l aufweist.

**Claims**

**1.** A film comprising at least one unimolecular layer of an amphiphilic compound, wherein the layer contains or comprises a compound of the formula (I)

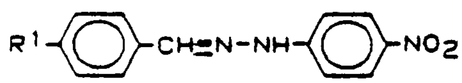

(I)

in which $R^1$ denotes the

$CH_3-(CH_2)_m-O-$ ,

$CH_3-(CH_2)_n-C(=O)-O-$ ,

$CH_3(CH_2)_nN(CH_2)_lH$ or

$CH_3(CH_2)_n(CO)N(CH_2)_lH$ radical and

m denotes a number from 10 to 25,
n denotes a number from 8 to 22 and 1 denotes a number from 0-25,
the molecules thereof being oriented in the same direction, parallel to one another and non-centrosymmetrically.

**2.** A film as claimed in claim 1, which comprises at least two unimolecular layers of different composition, in each case one layer containing or comprising a compound of the formula (I) alternating with a layer containing or comprising another amphiphilic compound.

**3.** A film as claimed in claim 2, which comprises at least two unimolecular layers of different composition, in each case one layer containing or comprising a compound of the formula (I) alternating with a layer containing or comprising an unsaturated amphiphilic compound.

**4.** A film as claimed in claim 3, wherein the unsaturated amphiphilic compound has the formula III

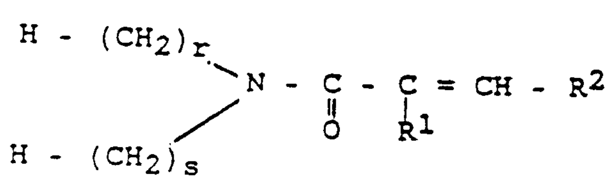

(III)

in which

$R^1$ denotes H, Cl, F, CN or $(CH_2)_tH$,
$R^2$ denotes H, $(CH_2)_uH$ or $-CH=CH-(CH_2)_uH$
r and s, independently of one another, denote a number from 0-22 and
t and u, independently of one another, denote a number from 0-24.

**5.** A film as claimed in claim 1, wherein the unimolecular layer additionally contains at least one second amphiphilic compound in a proportion of 10 to 90 mol-%, the sum of all amphiphilic compounds being 100% and $R^1$ in the formula I denoting $CH_3(CH_2)_nCO_2$-where n = 8-22.

**6.** A film as claimed in claim 5, wherein n is 10 to 14.

**7.** A film as claimed in claim 5, wherein the proportion of the second amphiphilic compound is 40 to 75 mol-%.

**8.** A film as claimed in claim 5, wherein the second amphiphilic compound contains at least one hydrophobic part containing at least 8 carbon atoms and at least one of the following polar groups: ether, hydroxyl, carboxylic acid, carboxylate, amine, carboxamide, ammonium salt, sulfate, sulfonic acid, phosphoric acid, phosphonic acid, phosphonate, phosphonamide, phosphoric acid ester or phosphoramide group.

**9.** A film as claimed in claim 8, wherein the second amphiphilic compound comprises at least one hydrophobic part containing at least 8 carbon atoms and at least one polar part which is selected from the following groups

$-OR^5$

$-COOR^3$

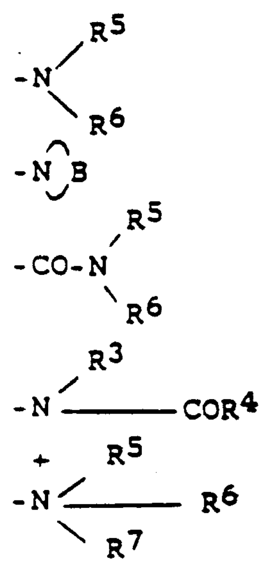

$-SO_3H$

$-OSO_3R^3$

$-OPO(OR^3)(OR^4)$

$-E$

$-O-E$

$-NR^3-E$,

where $R^3$ to R, B and E have the following meanings:

| | |
|---|---|
| $R^3$ and $R^4$, | independently of one another, denote H or $C_1$-$C_3$-alkyl, |
| $R^5$, $R^6$ and $R^7$, | independently of one another, denote H, $C_1$-$C_4$-alkyl, $-C_2H_4OH$ or $-CH_2-CHOH-CH_3$, in particular H or $CH_3$, |
| B | denotes a divalent organic radical, so that 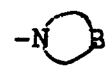 forms a nitrogen-containing heterocyclic ring, in particular a 5- or 6-membered saturated or unsaturated heterocyclic ring having 1 to 3 carbon atoms or N and O atoms or N and S atoms, and |
| E | 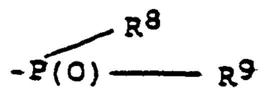 or 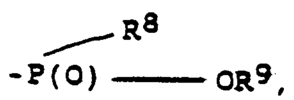 where $R^8$ and $R^9$, independently of one another, represent 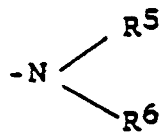 |

**10.** A film as claimed in claim 9, wherein the second amphiphilic compound is a fatty acid of the formula II

$CH_3(CH_2)_mCO_2H$ II,

where m denotes a number from 8 to 25, preferably 12 to 22.

**11.** A film as claimed in claim 8, wherein the second amphiphilic compound has the formula III

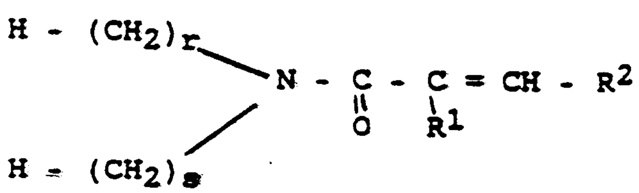

III

in which
$R^1$ denotes H, Cl, F, CN or $(CH_2)_tH$,
$R^2$ denotes H, $(CH_2)_uH$ or $-CH=CH(CH_2)_uH$,
r and s, independently of one another, denote a number 0-22, and
t and u, independently of one another, denote a number from 0-24.

**12.** A film as claimed in claim 11, wherein at least one of the values for r, s, t and u is at least 10.

**13.** A film as claimed in claim 12, wherein one of the values for t and u is at least 10 and the other value is a maximum of 1.

**14.** A film as claimed in claim 12, wherein the value for r is at least 10 and the values for t and u are a maximum of 1.

**15.** A film as claimed in claim 8, wherein the second amphiphilic compound has the formula IV

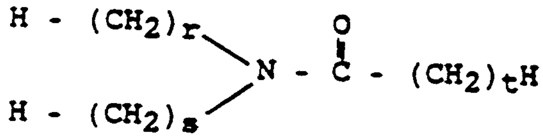

(IV)

where r and s, independently of one another, denote a number 0-22, and t denotes a number from 0-24, and at least one of the values for r, s and t is at least 8.

**16.** A film as claimed in claim 15, wherein at least one of the values for r, s and t is at least 10.

**17.** A film as claimed in claim 16, wherein the values for r and s are a maximum of 1 and the value for t is at least 10.

**18.** A film as claimed in claim 16, wherein the value for r is at least 10 and the value for t is a maximum of 1.

**19.** A film as claimed in claim 8, wherein the length of the all chain in the amphiphilic phenylhydrazone and of the hydrophobic part of the second amphiphilic compound employed differ by a maximum of 1 nm.

**20.** A film as claimed in claim 5, wherein the second amphiphilic compound has a water solubility at 20 ° C of less than 5 g/l.

## Revendications

**1.** Film constitué d'au moins une couche monomoléculaire elle-même constituée d'un composé amphiphile, caractérisé en ce que la couche contient un composé de formule (I)

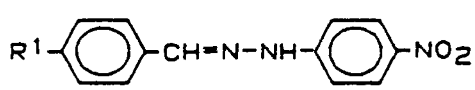

(I),

où $R^1$ est le radical

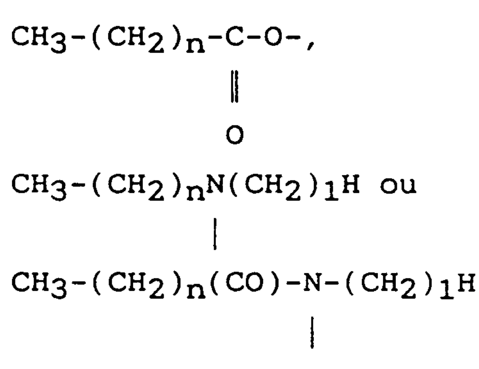

m est un nombre de 10 à 25,
n est un nombre de 8 à 22 et l est un nombre de 0-25,
ou est constituée de ce dernier, ces molécules étant orientées dans le même sens, parallèlement les unes aux autres et d'une manière non centro-symétrique.

**2.** Film selon la revendication 1, caractérisé en ce qu'il est constitué d'au moins deux couches monomoléculaires ayant des compositions différentes, chacune des couches contenant un composé de formule (I) ou constituée de cette dernière étant disposée en alternance avec une couche contenant un autre composé amphiphile ou constituée de cette dernière.

**3.** Film selon la revendication 2, caractérisé en ce qu'il est constitué d'au moins deux couches monomoléculaires ayant des compositions différentes, chacune des couches contenant un composé de formule (I) ou constituée de cette dernière étant disposée en alternance avec une couche contenant un composé amphiphile insaturé ou constitué de ce dernier.

**4.** Film selon la revendication 3, caractérisé en ce que le composé amphiphile insaturé a la formule générale (III)

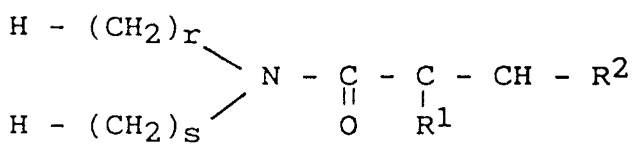

(III)

dans laquelle
$R^1$ est H, Cl, F, CN ou $(CH_2)_tH$
$R^2$ est H, $(CH_2)_uH$ ou $-CH=CH-(CH_2)_uH$
r et s, indépendamment l'un de l'autre, sont chacun un nombre de 0-22 et
t et u, indépendamment l'un de l'autre, sont chacun un nombre de 0-24

**5.** Film selon la revendication 1, caractérisé en ce que la couche monomoléculaire contient encore au moins un deuxième composé amphiphile selon une proportion molaire de 10 à 90 %, la somme de tous les composés amphiphiles est de 100 % et, dans la formule (I), $R^1$ est le radical $CH_3(CH_2)_n-CO_2-$, avec n = 8-22.

**6.** Film selon la revendication 5, caractérisé en ce que n vaut 10 à 14.

**7.** Film selon la revendication 5, caractérisé en ce que la proportion molaire du deuxième composé amphiphile est de 40 à 75 %.

**8.** Film selon la revendication 5, caractérisé en ce que le deuxième composé amphiphile contient au moins une partie hydrophobe, contenant au moins 8 atomes de carbone, let au moins l'un des groupes polaires suivants : éther, hydroxy, acide carboxylique, ester d'acide carboxylique, amine, amide d'acide carboxylique, sel d'ammonium, sulfate, acide sulfonique, acide phosphorique, acide phosphonique, ester de l'acide phosphonique, phosphonamide, ester de l'acide phosphorique ou phosphoramide.

**9.** Film selon la revendication 8, caractérisé en ce que le deuxième composé amphiphile est constitué d'au moins une partie hydrophobe ayant au moins 8 atomes de carbone et d'au moins une partie molaire choisie parmi les groupes suivants :

$-OR^5$

$-COOR^3$

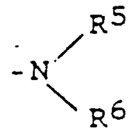

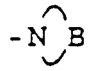

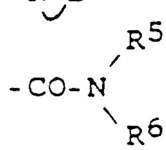

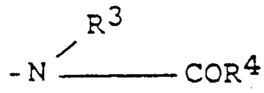

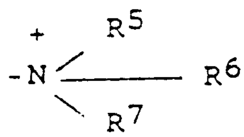

$-SO_3H$

$-OSO_3R^3$

$-OPO(OR^3)(OR^4)$

$-PO(OR^3)(OR^4)$

-E

-O-E

$-NR^3-E$,

où $R^3$ à $R^7$, B et E ont les significations suivantes :

$R^3$ et $R^4$, indépendamment l'un de l'autre, sont chacun H ou un radical alkyle en $C_1$-$C_3$,

$R^5$, $R^6$ et $R^7$, indépendamment les uns des autres, sont chacun H ou un radical alkyle en $C_1$-$C_4$, $-C_2H_4OH$ ou $-CH_2-CHOH-CH_3$, en particulier H ou $CH_3$

B est un radical organique divalent, de sorte que -N=B forme un hétérocycle azoté, en particulier un hétérocycle saturé ou insaturé à 5 ou 6 chaînons comportant 1 à 3 atomes de carbone ou atomes de N et O ou atomes de N et S, et

E est 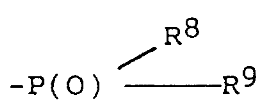 ou

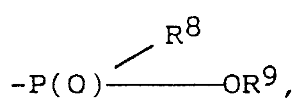,

où $R^8$ et $R^9$, indépendamment l'un de l'autre, représentent chacun

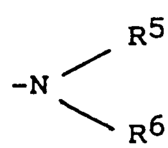

**10.** Film selon la revendication 9, caractérisé en ce que le deuxième composé amphiphile est un acide gras de formule générale (II)

$CH_3(CH_2)_mCO_2H$ (II)

dans laquelle m est un nombre de 8 à 25, de préférence de 18 à 22.

**11.** Film selon la revendication 8, caractérisé en ce que le deuxième composé amphiphile a la formule générale III

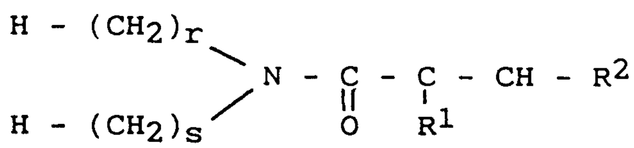 (III)

dans laquelle
$R^1$ est H, C1, F, CN ou $(CH_2)_tH$
$R^2$ est H, $(CH_2)_uH$ ou $-CH=CH(CH_2)_uH$,
r et s, indépendamment l'un de l'autre, sont chacun un nombre de 0-22 et
t et u, indépendamment l'un de l'autre, sont chacun un nombre de 0-24.

**12.** Film selon la revendication 11, caractérisé en ce qu'au moins l'une des valeurs de r, s, t et u vaut au moins 10.

**13.** Film selon la revendication 12, caractérisé en ce que l'une des valeurs de t et u est d'au moins 10, l'autre valeur étant au maximum égale à 1.

**14.** Film selon la revendication 12, caractérisé en ce que la valeur de r est d'au moins 10, et que les valeurs de t et u sont au maximum égales à 1.

**15.** Film selon la revendication 8, caractérisé en ce que le deuxième composé amphiphile a la formule générale (IV)

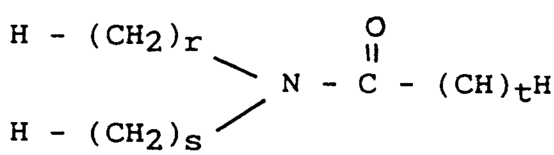 (IV)

dans laquelle r et s, indépendamment l'un de l'autre, sont chacun un nombre de 0-22 et t est un nombre de 0-24, et au moins l'une des valeurs de r, s et t est d'au moins 8.

**16.** Film selon la revendication 15, caractérisé en ce qu'au moins l'une des valeurs de r, s et t est au moins égale à 10.

**17.** Film selon la revendication 16, caractérisé en ce que les valeurs de r et s sont au maximum égales à 1, la valeur de t étant au moins égale à 10.

**18.** Film selon la revendication 16, caractérisé en ce que la valeur de r est d'au moins 10 et que la valeur de t est au maximum égale à 1.

**19.** Film selon la revendication 8, caractérisé en ce que la longueur de la chaîne alkyle de la phénylhydrazone amphiphile utilisée, et celle de la partie hydrophobe du deuxième composé amphiphile, s'écartent l'une de l'autre d'une valeur maximale de 1 nm.

**20.** Film selon la revendication 5, caractérisé en ce que le deuxième composé amphiphile a à 20 ° C une solubilité dans l'eau inférieure à 5 g/l.